# EUROPÄISCHE PATENTSCHRIFT

(11) **EP 2 556 046 B1**
(45) Veröffentlichungstag und Bekanntmachung des Hinweises auf die Patenterteilung: **08.10.2014**
(21) Anmeldenummer: 11712274.7
(22) Anmeldetag: 06.04.2011
(51) Int. Cl.: C07C 209/26, C07C 211/08, C07C 211/17, C07C 211/27

(54) **VERFAHREN ZUR HERSTELLUNG VON UNSYMMETRISCHEN SEKUNDÄREN TERT-BUTYLAMINEN IN DER FLÜSSIGPHASE**
PROCESS FOR PREPARING UNSYMMETRICAL SECONDARY TERT-BUTYLAMINES IN THE LIQUID PHASE
PROCÉDÉ DE FABRICATION DE TERT-BUTYLAMINES SECONDAIRES ASYMÉTRIQUES EN PHASE LIQUIDE

(30) Priorität: 07.04.2010 EP 10159254
(43) Veröffentlichungstag der Anmeldung: 13.02.2013
(73) Patentinhaber: BASF SE, 67056 Ludwigshafen (DE)
(72) Erfinder: WIGBERS, Christof, Wilhelm, 68167 Mannheim (DE); MÜLLER, Christoph, 68165 Mannheim (DE); MELDER, Johann-Peter, 67459 Böhl-Iggelheim (DE); STEIN, Bernd, 64665 Alsbach-Hähnlein (DE); MEISSNER, Harald, 67454 Haßloch (DE); HADERLEIN, Gerd, 67269 Grünstadt (DE); GUTFRUCHT, Norbert, 67466 Lambrecht (DE)
(86) Internationale Anmeldenummer: PCT/EP2011/055372
(87) Internationale Veröffentlichungsnummer: WO 2011/124619

(56) Entgegenhaltungen:
- WEINSTOCK, LEONARD M. ET AL: "Synthesis of the .beta.-adrenergic blocking agent timolol from optically active precursors", JOURNAL OF ORGANIC CHEMISTRY, 41(19), 3121 -4 CODEN: JOCEAH; ISSN: 0022-3263, 1976, XP002634073,
- CHEN, ZHENLIANG ET AL: "The synthesis of baclofen and GABOB via Rh(II) catalyzed intramolecular C-H insertion of .alpha.-diazoacetamides", TETRAHEDRON, 61(6), 1579 -1586 CODEN: TETRAB; ISSN: 0040-4020, 2005, XP025382974,
- "Houben-Weyl, Methoden der organischen Chemie", 1957, Georg Thieme Verlag, Stuttgart, XP002634072, Bd. 11/1, Seiten 618-620, in der Anmeldung erwähnt Seite 618, siebtletzte Zeile - Seite 619, Zeile 8 Seite 620, Zeile 13 - Zeile 20

## Beschreibung

Die vorliegende Erfindung betrifft ein Verfahren zur Herstellung von unsymmetrischen sekundären tert-Butylaminen, die neben dem tert.-Butylrest noch einen Alkyl-, Cycloalkyl- oder Benzylrest enthalten. Ihre Herstellung erfolgt durch Umsetzung entsprechender Aldehyde mit tert.-Butylamin und Wasserstoff in Gegenwart von Hydrierkatalysatoren (reduktive Aminierung) in der Flüssigphase.

Sekundäre Amine stellen wichtige, technisch genutzte Stoffe dar. Sie dienen beispielsweise als Polymerisations- und Härtungskatalysatoren für die Herstellung von Kunststoffformkörpern auf der Basis von Epoxiden und Polyurethanen, als Korrosionsinhibitoren und als Ausgangsstoffe für Flockungsmittel und Detergenzien. Weiterhin werden sekundäre Amine als Zwischenprodukte im Pflanzenschutz verwendet.

In WO 2009/084538 werden unsymmetrische Amine mit tert.-Butyl- und Alkylresten zur Herstellung von Vulkanisationsbeschleunigern für Kautschuk beschrieben.

Sekundäre Amine sind durch Alkylierung von primären Aminen mit Alkylhalogeniden, durch Acetylierung von primären Aminen und anschließende Reduktion der Carbonylgruppe mit Lithiumaluminiumhydrid und durch reduktive, insbesondere hydrierende Aminierung von Aldehyden mit primären Aminen zugänglich.

Dies gilt prinzipiell auch für tert.-Butylgruppen enthaltende unsymmetrische sekundäre Amine:
L. Weinstock et al. beschreibt in Journal of Organic Chemistry, 1976, 41, Seiten 3121 bis 3124 ein Verfahren zur Herstellung eines unsymmetrischen sekundären tert.-Butylamins durch hydrierende Aminierung von mit 2 Hydroxygrupppen substituierten Glyceraldehyd mit tert.-Butylamin.
Z. Chen et al. beschreibt in Tetrahedron, 2005, 61, Seiten 1579 bis 1586 ebenfalls ein Verfahren zur Herstellung eines unsymmetrischen sekundären tert.-Butylamins durch hydrierende Aminierung eines Aldehyds mit tert.-Butylamin, wobei der Aldehyd zu Beginn der Reaktion vorgelegt wird.
J. C. Bottaro et al. beschreibt in Journal of Organic Chemistry, 1991, 56, Seiten 1305 bis 1307, dass Ethyl-tert.-butylamin durch Umsetzung von tert.-Butylamin mit Ethylbromid im Molverhältnis 3 : 1 mit 85 %iger Ausbeute herstellbar ist. Nachteilig an diesem Verfahren ist, dass der anfallende Bromwasserstoff nach Neutralisation zu Salzanfall führt, dass überschüssiges tert.-Butylamin aus wirtschaftlichen Gründen abgetrennt und zurückgeführt werden muss und dass Korrosionsprobleme auftreten.
M. Newcomb et al. beschreibt hingegen in Journal of the American Chemical Society, 1990, 112, Seiten 5186 bis 5193 tert.-Butylamin mit Acetanhydrid zu acetylieren (Ausbeute 40 %) und das erhaltene N-tert.-Butylacetamid mit Lithiumaluminiumhydrid zu Ethyl-tert.-butylamin zu reduzieren. Das Verfahren ist jedoch zweistufig, ermöglicht nur niedrige Ausbeuten und ist durch den Anfall von sauerstoffhaltigen Aluminiumverbindungen belastet.
Yu. Smirnow et al. beschreibt in Zhurnal Organicheskoi Khimii (1992), 28 (3), Seiten 461 bis 467, dass Ethyl-tert.-butylamin auch durch elektrochemische reduktive Aminierung von Acetaldehyd mit tert.-Butylamin an Bleikathoden in 60 %iger Ausbeute herstellbar ist. Nachteilig sind vor allem die niedrigen Ausbeuten.

Die hydrierende Aminierung von Aldehyden mit primären Aminen in Gegenwart von Hydrierkatalysatoren zu entsprechenden unsymmetrischen sekundären Aminen ist ebenfalls bekannt. Hierbei muss wie bereits in GB-A 1.116.610, Seite 2, Spalte 1, Zeilen 1 bis 4 beschrieben, deren Weiterreaktion mit den jeweiligen Aldehyden zu unerwünschten tertiären Aminen so weit wie möglich zurückgedrängt werden, um hohe Ausbeuten an sekundären Aminen erzielen zu können. Außerdem gilt es, Reaktionsbedingungen und Hydrierkatalysatoren zu finden, unter denen die Nebenreaktionen - Aldehyd-Decarbonylierung, Aldehyd-Hydrierung zu Alkoholen und Aldolkondensation zwischen zwei Aldehydmolekülen - weitestgehend unterbleiben.

In Advanced Synthesis & Catalysis, 2002, 344, Seite 1041, Kapitel 3.1, erster Absatz, wird beschrieben, dass bei der Umsetzung eines Amins mit einer Carbonylverbindung Ausbeuten und Selektivitäten in hohem Maße von der sterischen Hinderung der Ausgangsverbindungen abhängen. Diese sterische Hinderung spielt auch eine Rolle im Bereich der Aminfunktion wie in Kapitel 3.1, dritter Absatz und Schema 10 erläutert. So entsteht bei der reduktiven Aminierung von Aceton mit 2.4.6-Trimethylanilin das sekundäre Amin nur in 36 %iger, mit Anilin dagegen mit 98 %iger Ausbeute. Eine verringerte Reaktionsgeschwindigkeit der reduktiven Aminierung kann auch dazu führen, dass die Carbonylverbindung in höherem Maße zum entsprechenden Alkohol hydriert wird. Die im Stand der Technik beschriebenen Verfahren beschreiben daher nie den Einsatz von sterisch gehindertem tert.-Butylamin, da hierbei von geringeren Ausbeuten an dem erfindungsgemäßen sekundären tert-Butylamin I und größeren Mengen an Alkohol durch die Hydrierung des jeweiligen Aldehyds ausgegangen werden muss.

In DE-A 101 22 758 wird ein Verfahren zur Herstellung von unsymmetrischen sekundären Aminen beschrieben, bei dem man Aldehyd und primäres Amin in der Flüssigphase getrennt von einander, aber gleichzeitig in den Reaktor einspeist und dort mit Wasserstoff an einem Nickel enthaltenden Katalysator umsetzt. Das Verfahren ist besonders zur Herstellung von N-Ethyl-n-propylamin, wie in Beispiel 4 an Hand der gaschromatographischen Ausbeute von 92 % zu erkennen ist, bezogen auf Propionaldehyd, geeignet. Nachteilig ist dabei, wie bereits das Beispiel 1 in DE-A 101 22 758 zeigt, dass zur kontinuierlichen Herstellung von N-Ethyl-n-propylamin bei hohem Druck um die 8 MPa und einem fünffachen molaren Überschuss an Ethylamin, bezogen auf Propionaldehyd, gearbeitet werden muss. Dementsprechend muss eine große Menge an Ethylamin destillativ abgetrennt und in die Hydrierung zurückgeführt werden. Des Weiteren ist in dieser Offenbarung die Herstellung von sekundären tert-Butylaminen nicht beschrieben.

In Houben/Weyl, Methoden der organischen Chemie, vierte Auflage, Band XI/1, Stickstoffverbindungen II, Georg-Thieme-Verlag, 19597, Seite 620, wird die Herstellung von n-Butyl-i-butylamin beschrieben. Hierzu werden in einem Rührautoklaven i-Butylamin, Raney-Nickel und Methanol als Lösungsmittel vorgelegt. Bei 100°C und einem Wasserstoffdruck von 100 bar werden etwa äquimolare Mengen n-Butyraldehyd, bezogen auf vorgelegtes Amin, kontinuierlich innerhalb von 65 Minuten zugeführt. Nach destillativer Aufarbeitung wurde eine n-Butyl-isobutylamin-Ausbeute von 86 % erzielt. Nachteilig an diesem Verfahren sind der hohe Druck und die Verwendung eines Lösungsmittels, das abgetrennt und zurückgeführt werden muss.

Andererseits ist von Chem. Ind. Dekker in Catalysis of Organic Reactions, 75, Seiten 266 bis 270, 1998 die Herstellung von N-Ethyl-n-butylamin beschrieben. Auch hier wird in einem Autoklaven das Amin, in diesem Fall Ethylamin, zusammen mit dem Katalysator vorgelegt, um anschließend bei 80°C und 24 bar über einen Zeitraum von 4,5 bis 5 Stunden n-Butyraldehyd hinzuzufügen. Im Gegensatz zur Offenbarung in Houben/Weyl beträgt das Molverhältnis von Amin zu Aldehyd hierbei jedoch 2,6 zu 1. Trotzdem werden in Gegenwart von Nickel-Katalysatoren Reaktionsausträge erhalten, die nur rund 64 % N-Ethyl-n-butylamin enthalten. Mit Edelmetallkatalysatoren (5 % Pd, 5 % Pt, 5 % Rh, 5 % Ru auf Aktivkohle) betragen die N-Ethyl-n-butylamingehalte der Reaktionsausträge jedoch 82 bis 96 %.Nachteilig bei diesem Verfahren sind die niedrigen Wertproduktausbeuten in Gegenwart von Nickel-Katalysatoren und die Notwendigkeit, überschüssiges Amin abzutrennen und zurückzuführen. Auch hierbei wird die Herstellung von sekundären tert-Butylaminen I nicht beschrieben.

Auch in GB-A 1.116.610 wird Amin in einem Reaktor zusammen mit einem Hydrierkatalysator vorgelegt und in Gegenwart von Wasserstoff mit einem Aldehyd versetzt. N-Methylbutylamin kann so gemäß Beispiel 3 bei 100°C und 30 bar aus n-Butyraldehyd und Methylamin in Gegenwart von Raney-Nickel in 97 %iger Ausbeute hergestellt werden. Das Molverhältnis von Methylamin zu n-Butyraldehyd beträgt 1,25 zu 1. Nachteilig an diesem Verfahren ist, dass zur Vermeidung der Bildung von tertiären Aminen große Mengen an rund 3 %iger wässriger Natronlauge zugesetzt und nach der Umsetzung wieder abgetrennt werden müssen. Die Herstellung von sekundären tert-Butylaminen I wird auch hier nicht explizit beschrieben.

Die Reaktionsbedingungen und die Ergebnisse der reduktiven Aminierungen in den vier vorangehenden Dokumenten sind in der nachfolgenden Tabelle 1 zusammengefasst.

**Tabelle 1:**

| Literaturstelle | prim. Amin | Aldehyd | Molverh. Aldehyd zu Amin | Katalysator | Zugabe | Zugabezeit | Druck [bar] | Lösungsmittel bzw. Zusatz | Ausbeute [%] |
|---|---|---|---|---|---|---|---|---|---|
| DE 10122758 | Ethylenamin | Propionaldehyd | 1:5 | Ni | Gleichzeitige Zugabe beider Reaktanden | - | 80 | - | 92 |
| Catalysis of Org. Reactions | Wässr. Methylamin (70 % ig) | n-Butyraldehyd | 1:2,6 | Ni | Amin vor-gelegt | 5 | 24 | - | 64 |
| | | | | 5 % Pd/C | | | | - | 83 |
| Houben-Weyl | i-Butylamin | n-Butryaldehyd | 1:1 | Ra-Ni | | 1 | 100 | CH₃OH | 86 |
| GB 1116610 Bsp.2 | Methylamin | n-Butryaldehyd | 1:1,25 | Ra-Ni | | 5 | 100 | ca. 3 %ige NaOH | 97 |

Es bestand daher die Aufgabe, ein Verfahren zur reduktiven Aminierung von Aldehyden mit tert.-Butylamin in Gegenwart von Hydrierkatalysatoren bereitzustellen, das die oben genannten Nachteile vermeidet. Das Verfahren sollte hohe Ausbeuten an unsymmetrischen sekundären Aminen liefern, ohne größere Mengen an nicht umgesetzten Ausgangsprodukten abtrennen und in das Verfahren zurückführen zu müssen. Außerdem sollte bei möglichst niedrigen Drücken gearbeitet werden können. Um die Nebenproduktbildung zu minimieren, sollten niedrige Temperaturen angewandt werden. Die Aufarbeitung sollte einfach sein und zu einer hohen Produktreinheit führen.

Diese Aufgabe wird gelöst durch ein Verfahren zur Herstellung von unsymmetrischen sekundären tert-Butylaminen der Formel I durch reduktive Aminierung von Aldehyden der Formel II mit tert-Butylamin und Wasserstoff in der Flüssigphase in Gegenwart von Hydrierkatalysatoren, umfassend folgende Schritte:
(i) Bereitstellung von tert-Butylamin und dem Hydrierkatalysator in einem Druckgefäß,
(ii) Zugabe von Wasserstoff und kontinuierliche Zugabe eines Aldehyds der Formel II

   R-CHO II,

   wobei der Aldehyd der Formel II ausgewählt ist aus der Gruppe von Acetaldehyd, Propionaldehyd, n-Butyraldehyd, i-Butyraldehyd, sekundärer Butyraldehyd, Pivalinaldehyd, n-Pentanal, n-Hexanal, 2-Ethylhexanal, 2-Methylpentanal, 3-Methylpentanal, 4-Methylpentanal, n-Octanal, n-Decanal, n-Undecanal, n-Dodecanal, 11-Methyldodecanal, Cyclopentylaldehyd. Cyclohexylaldehyd, Cycloheptvlaldehyd, Adamantylaldehyd, Phenylacetaldesyd und Benzaldehyd oder Gemische dieser Aldehyde
   und das Verhältnis von Wasserstoff zu Aldehyd der Formel II mindestens äquimolar ist,
(iii) Halten der Temperatur während der Zugabe des Aldehyds in Schritt (ii) im Bereich von 50 bis 150 °C und Halten des Gesamtdrucks während der Zugabe des Aldehyds in Schritt (ii) im Bereich von 2 bis 120 bar,
(iv) Entwässerung des Hydrieraustrag aus Schritt (iii), der das gebildete sekundäre tert-Butylamin der Formel I 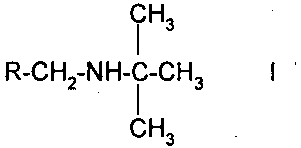 und das Reaktionswasser enthält und
(v) anschließende fraktionierte Destillation des entwässerten Hydrieraustrages aus Schritt (iv).

Die erfindungsgemäße Umsetzung lässt sich durch die folgende Formelgleichung beschreiben.

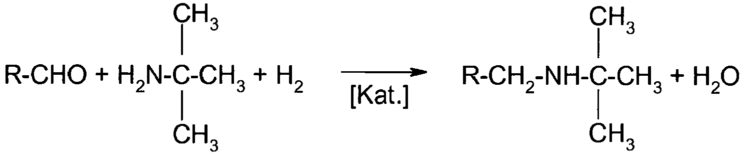

Die reduktive Aminierung wird in der Flüssigphase durchgeführt. Dies bedeutet, dass tert.-Butylamin, mit einem Siedepunkt von 44°C bei einem Druck von1013 mbar, dem Druckgefäß in flüssiger Form zugeführt werden kann.

Es kann vorteilhaft sein, die reduktive Aminierung in Gegenwart eines unter den Reaktionsbedingungen inerten Lösungsmittels durchzuführen. Unter inert ist zu verstehen, dass das Lösungsmittel nicht als Reagenz an der Reaktion teilnimmt. Hierfür kommen beispielsweise N-Methylpyrrolidon oder Ether wie Tetrahydrofuran, Dioxan, Ethylenglykoldimethylether in Frage. Dabei kann das Lösungsmittel zusammen mit dem tert.-Butylamin und dem Katalysator vorgelegt, mit dem Aldehyd II zugeführt oder auf das vorgelegte Reaktionsgemisch und den zugeführten Aldehyd II verteilt werden. Bevorzugt ist jedoch, in Abwesenheit eines Lösungsmittels zu arbeiten.

Als Katalysatoren für die aminierende Hydrierung können alle dem Fachmann bekannten Hydrierkatalysatoren verwendet werden. Diese sind in Houben-Weyl, Methoden der organischen Chemie, 4. Auflage, Band 11/1, Seite 602, und Handbook of Heterogeneous Catalysis, 2. Auflage, Band 7, 2008, Wiley VCH, Seite 3554, beschrieben. Als Hydrierkatalysatoren sind bevorzugt die Metalle und/oder Metalloxide ausgewählt aus der Gruppe von Nickel, Kobalt, Ruthenium, Rhodium, Palladium, Platin, Kupfer, insbesondere die Metalle aus der Gruppe von Palladium, Kobalt, Nickel und Ruthenium, und Gemische dieser Metalle. Kobalt, Nickel und Kupfer können auch als Raney-Katalysatoren verwendet werden. Besonders bevorzugt sind Palladium, Kobalt und Ruthenium. Ganz besonders bevorzugt ist Palladium als Hydrierkatalysator, mit dem sich besonders gute Ausbeuten für die erfindungsgemäße Umsetzung erzielen lassen. Die Metalle können als solche verwendet werden, aber auch auf Träger aufgebracht sein. Bevorzugte Träger sind dabei ausgewählt aus der Gruppe von Aluminiumoxid, Siliziumdioxid, Titandioxid, Zirkondioxid und Aktivkohle. Besonders bevorzugt sind die geträgerten Metalle. Bevorzugte Träger sind Aktivkohle, Aluminiumoxid und Titandioxid. Als Träger ist Aktivkohle ganz besonders bevorzugt. Insbesondere ganz besonders bevorzugt ist als Hydrierkatalysator Palladium auf Aktivkohle (Pd/Kohle).

Die Menge an Hydriermetall (ohne Katalysatorträger) beträgt 0,001 bis 10 Gew.-%, bevorzugt 0,1 bis 5 Gew.-%, bezogen auf die insgesamt verwendete Masse aus tert.-Butylamin.

Die Hydrierkatalysatoren werden in dem Druckgefäß mit tert.-Butylamin und gegebenenfalls dem Lösungsmittel vorgelegt und durch Rühren mit dem zugeführten Aldehyd II intensiv vermischt. Die Zuführung des Aldehyds der Formel II zu dem im Reaktionsgefäß vorgelegten tert.-Butylamin und dem Hydrierkatalysator sowie dem gegebenenfalls vorliegenden Lösungsmittel erfolgt so, dass eine gleichmäßige Durchmischung der Reaktanten möglich ist. Bevorzugt erfolgt die Zugabe des Aldehyds der Formel II innerhalb von 30 bis 600 Minuten, besonders bevorzugt innerhalb von 120 bis 480 Minuten, insbesondere bevorzugt innerhalb von 300 bis 420 Minuten. Bei der Zugabe des Aldehyds II kann es vorteilhaft sein, die Dosiergeschwindigkeit nach Zugabe von 2/3 der Gesamtmenge an Aldehyd II um 50% zu reduzieren (Dosierrampe). Nach vollständiger Zugabe des Aldehyds der Formel II ist es bevorzugt, noch eine Nachrührzeit im Bereich von 0,5 bis 12 Stunden, besonders bevorzugt von 0,5 bis 2 Stunden unter den gewählten Bedingungen einzuhalten.

Als Druckgefäß sind jede dem Fachmann bekannten Hydrierrührreaktoren geeignet, in denen der Katalysator suspendiert und/oder fest angeordnet werden kann. Bevorzugt sind Hydrierrührreaktoren, die Katalysatorkörbe enthalten.

Für das erfindungsgemäße Verfahren können als Aldehyde der Formel II vorzugweise Acetaldehyd, Propionaldehyd, n-Butyraldehyd, i-Butyraldehyd, sekundärer Butyraldehyd, Pivalinaldehyd, n-Pentanal, n-Hexanal, 2-Methylpentanal, 3-Methylpentanal, 4-Methylpentanal, Cyclohexylaldehyd oder Gemische dieser Verbindungen eingesetzt werden. Insbesondere bevorzugt sind Acetaldehyd, Propionaldehyd, Butyraldehyd, Cyclohexanaldehyd oder Benzaldehyd. Ganz besonders bevorzugt ist Acetaldehyd.

Da Nebenreaktionen wie die Decarbonylierung und die Aldolkondensation der eingesetzten Aldehyde der Formel II und die Bildung von tertiären Aminen der Formel III 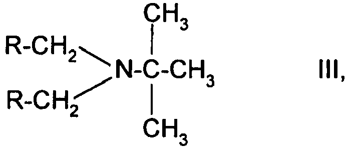 mit steigender Temperatur zunehmen, wird das erfindungsgemäße Verfahren bei möglichst niedrigen Temperaturen durchgeführt. Unter niedrigen Temperaturen sind dabei Temperaturen unterhalb von 150°C gemeint. Bevorzugt wird im Bereich von 50 bis 150 °C, besonders bevorzugt im Bereich von 60 bis 130 °C, ganz besonders bevorzugt im Bereich von 70 bis 125 °C gearbeitet. Wenn als Aldehyde der Formel II aromatische Aldehyde mit substituierten oder unsubstituierten Phenylrest oder Phenylalkylrest als Rest R verwendet werden, wird die Reaktion bevorzugt bei mindestens 110 °C innerhalb des erfindungsgemäßen Temperaturbereichs durchgeführt um die Ausbeuten zu optimieren.

Bei der aminierenden Hydrierung müssen pro Mol Aldehyd der Formel II mindestens äquimolare Mengen an Wasserstoff vorhanden sein.

Der Gesamtdruck im Reaktionsgefäß bei der jeweiligen Temperatur setzt sich aus den Partialdrücken der Einsatzstoffe und der Reaktionsprodukte, also Wasserstoff, tert.-Butylamin, Aldehyd II, Amin I, Wasser und gegebenenfalls einem mitverwendeten Lösungsmittel zusammen. Durch Aufpressen von Wasserstoff wird der Druck auf den gewünschten Reaktionsdruck erhöht. Um den Verbrauch an Wasserstoff auszugleichen, wird der Gesamtdruck während der Reaktionszeit durch Nachpressen von Wasserstoff konstant gehalten.

Der Gesamtdruck beträgt 2 bis 120 bar, bevorzugt 3 bis 50 bar, sehr bevorzugt 4 bis 20 bar, besonders bevorzugt 5 bis 10 bar.

Das Molverhältnis von tert.-Butylamin zu Aldehyd der Formel II sollte nicht größer als 1 zu 1,4 sein. Bevorzugt ist ein Verhältnis von tert-Butylamin zu Aldehyd der Formel II im Bereich von 1 zu 1,3, besonders bevorzugt 1 zu 1,2, ganz besonders bevorzugt 1 zu 1,1 und insbesondere ganz besonders bevorzugt 1 zu 1.

Das erfindungsgemäße Verfahren ermöglicht die Herstellung von sekundärem Amin der Formel I in hoher Ausbeute. Gleichzeitig wird die Bildung von tertiärem Amin der Formel III aber auch von anderen möglichen Nebenprodukten der Reaktion weitgehend vermieden. Das erfindungsgemäße Verfahren weist somit neben der hohen Ausbeute auch eine hohe Selektivität für die Herstellung des sekundären Amins der Formel I auf.

Nach erfolgter Hydrierung gemäß Schritt (ii) und (iii) des erfindungsgemäßen Verfahrens wird der Hydrierkatalysator, falls er suspendiert war, nach Abkühlen und Entspannung des Hydrieraustrags durch Filtration, Zentrifugieren oder Querstromfiltration abgetrennt. War der Katalysator fest angeordnet, erfolgt die Abtrennung des Hydrieraustrages durch Entfernung aus dem Reaktor. Der Katalysator kann in die nächste halbkontinuierliche Hydrierung zurückgeführt werden. Dabei kann es notwendig sein, Verluste an ursprünglicher Katalysatormenge durch Abrieb und/oder Desaktivierung durch Zugabe von frischem Katalysator zu ergänzen.

Der von Katalysator befreite Hydrieraustrag enthält neben dem erfindungsgemäßen Amin der Formel I als Nebenprodukte geringe Mengen an tertiären Aminen der Formel III.

Weiterhin sind gegebenenfalls geringe Mengen an Alkoholen R-CH₂OH, die durch Reduktion von Aldehyden II gebildet wurden, an tert.-Butylamin und an Aldehyd II enthalten. Unter geringen Mengen sind dabei jeweils weniger als 5 Gew.-%, bevorzugt weniger als 3 Gew.-%, besonders bevorzugt weniger als 1 Gew.-% der genannten Verbindungen, bezogen auf den katalysatorfreien Hydrieraustrag zu verstehen.

Bei der reduktiven Aminierung entstehen etwa 5 bis 20 Gew.-% Wasser, bezogen auf den katalysatorfreien Hydrieraustrag. Die sekundären unsymmetrischen tert-Butylamine I bilden mit Wasser Azeotrope. Daher lassen sich destillativ nur Gemische, bestehend aus Reaktionswasser und Amin I aus dem Hydrieraustrag abtrennen.

In EP-B 1312599 und EP-B 1312600 ist die Auftrennung von aminhaltigen Gemischen beschrieben, die ein oder mehrere Amine, Wasser, Leichtsieder und Schwersieder enthalten. Die Auftrennung eines sekundäres tert.-Butylaminhydriergemisches wird jedoch nicht beschrieben. Die Auftrennung erfolgt durch
(a) die destillative Abtrennung von Leichtsiedern von dem aminhaltigen Gemisch,
(b) gegebenenfalls destillative Abtrennung von Schwersiedern von dem aminhaltigen Gemisch,
(c) Extraktion des aminhaltigen Gemischs mit Natronlauge unter Gewinnung einer wässrigen, Natronlauge enthaltenden ersten Phase und einer wässrig-organischen, Amin enthaltenden zweiten Phase,
(d) Destillation der wässrig-organischen zweiten Phase aus Schritt (c) unter Gewinnung von Amin/Wasser-Azeotrop und von im wesentlichen wasserfreien Amin, und Rückführung des Amin/Wasser-Azeotrops in den Extraktionsschritt (c).

Das im Wesentlichen wasserfreie Amin muss destillativ weiter gereinigt werden. In einem Ausführungsbeispiel werden die Teilschritte der Aufarbeitung an einem Hydrieraustrag demonstriert, der durch reduktive Aminierung von 1,5-Pentandiol mit Ammoniak unter Bildung von Piperidin erhalten wurde.

Im erfindungsgemäßen Verfahren kann die Aufarbeitung des katalysatorfreien Hydrieraustrags auch durch Destillation und Brechung der Amin/Wasser-Azeotrope mit Natronlauge erfolgen. Destillation und Brechung des Amin/Wasser-Azeotrops können kontinuierlich, bevorzugt diskontinuierlich, durchgeführt werden.

Wenn der katalysatorfreie Hydrieraustrag durch Destillation und Brechung der Azeotrope aufgearbeitet werden soll, so erfolgt im Gegensatz zum Stand der Technik als erstes die Brechung des Amin I/Wasser-Azeotrops durch Behandlung des katalysatorfreien Hydrieraustrags mit wässriger, beispielsweise 50 %iger wässriger Natronlauge. Die Natronlaugekonzentration in der wässrigen Lösung kann 1 bis 75 Gew.-%, bevorzugt 25 bis 50 Gew.-%, betragen. Anstelle von Natronlauge können auch andere Alkali- und Erdalkalihydroxide verwendet werden. Nach Extraktion des Hydrieraustrags mit der wässrigen Natronlauge wird diese durch Phasentrennung abgetrennt. Der Restwassergehalt der organischen Phase kann zum Beispiel durch Karl-Fischer-Titration ermittelt werden. Die für die Wasserabtrennung benötigte Menge an Natronlauge lässt sich durch wenige Vorversuche bestimmen.

Die für die Extraktion mit Natronlauge eingesetzte Extraktionsvorrichtung kann einoder mehrstufig ausgebildet sein, beispielsweise ein einzelner Mixer-Settler-Extraktor. Mehrstufige Extraktionen sind beispielsweise Extraktionskolonnen oder Extraktionskaskaden. Als Extraktionskolonnen eignen sich beispielsweise Füllkörper-, Siebboden-, Kaskaden-, Pulsations-, Rotations- und Zentrifugalkolonnen. Eine Extraktionskaskade sind beispielsweise mehrere hintereinander geschaltete Mixer-Settler-Extraktoren, die auch platzsparend als Turmextraktor oder Kastenextraktor ausgeführt sein können. Ist der Extraktor mehrstufig, so ist bevorzugt eine Gegenstrom-Extraktionskolonne mit im allgemeinen 1 bis 25, bevorzugt 4 bis 10 theoretischen Trennstufen bevorzugt. Diese wird im allgemeinen bei einem Druck betrieben, bei dem alle Komponenten des Extraktionsgemisches unterhalb ihres Siedepunktes vorliegen, und sich ferner eine Viskosität der beiden Phasen einstellt, bei der eine Dispergierung der beiden Phasen problemlos möglich ist. Die Temperatur beträgt im Allgemeinen im Bereich von 5 bis 200°C, bevorzugt im Bereich von 20 bis 70°C, besonders bevorzugt im Bereich von 40 bis 50°C. Nach Phasentrennung wird die wässrige, Natronlauge enthaltende Phase als Abstrom aus dem Verfahren ausgeschleust.

Falls die abgetrennte wässrige Natronlauge wesentliche Mengen an sekundärem tert.-Butylamin der Formel I, Aldehyd der Formel II und/oder tert.-Butylamin enthält, so können diese Verbindungen durch Extraktion mit organischen Lösungsmitteln zurückgewonnen werden. Unter wesentlichen Mengen ist dabei mehr als 10 Gew.-%, bevorzugt mehr als 5 Gew.-%, besonders bevorzugt mehr als 2 Gew.-%, bezogen auf den wasser- und katalysatorfreien Hydrieraustrag, zu verstehen.

Als organische Lösungsmittel kommen dabei zum Beispiel aliphatische, cycloaliphatische oder aromatische Kohlenwasserstoffe in Frage, die mit wässriger Natronlauge eine Mischungslücke besitzen. Beispiele für derartige Kohlenwasserstoffe sind n-Hexan, n-Octan, Cyclohexan, Toluol und Ethylbenzol oder Gemische dieser Verbindungen.

Die wässrige Natronlaugephase wird von der Kohlenwasserstoffphase durch Phasentrennung abgetrennt. Aus der Kohlenwasserstoffphase wird der Kohlenwasserstoff destillativ entfernt. Das zurückgewonnene sekundäre tert-Butylamin der Formel I, der Aldehyd der Formel II und/oder tert.-Butylamin kann mit der Hauptmenge an rohem sekundären tert-Butylamin der Formel I vereinigt und destillativ aufgereinigt werden.

Es ist weiterhin möglich, das Azeotrop aus sekundärem tert.-Butylamin der Formel I und Wasser durch Zugabe von Kohlenwasserstoffen zum katalysatorfreien Hydrieraustrag, durch anschließende Abdestillierung von Kohlenwasserstoff/Wasser-Heteroazeotropen aus dem Hydrieraustrag, dann Abtrennen der Wasser- von der Kohlenwasserstoffphase und Rückführung der Kohlenwasserstoffphase in die Destillation zu brechen.

Eine weitere Möglichkeit besteht darin, zunächst das Azeotrop aus sekundärem tert-Butylamin der Formel I und Wasser destillativ abzutrennen und dann erst die Entwässerung durch Natronlauge-Behandlung oder Destillation mit Kohlenwasserstoffen durchzuführen.

Schließlich lässt sich die Wasserentfemung durch Natronlaugebehandlung mit der Wasserentfernung durch Destillation mit Kohlenwasserstoffen verknüpfen. Hierbei entfernt man die Hauptmenge an Wasser im Hydrieraustrag durch Natronlaugebehandlung, beispielsweise durch einstufige Extraktion mit Natronlauge, trennt die Phasen, extrahiert die Natronlaugephase mit einem Kohlenwasserstoff, trennt die Kohlenwasserstoffphase von der Natronlaugephase, vereinigt die abgetrennte Kohlenwasserstoffphase mit dem katalysatorfreien Hydrieraustrag und entfernt das noch vorhandene Wasser oder einen Teil davon durch Azeotropdestillation.

In einem besonders bevorzugten Verfahren wird das Wasser vor der destillativen Aufarbeitung des Hydrieraustrags nicht vollständig abgetrennt. Der Wassergehalt des Hydrieraustrags darf beispielsweise unter 5 Gew.-%, bevorzugt unter 3 Gew.-%, besonders bevorzugt unter 0,9 Gew.-% betragen. Liegt nur wenig Restwasser vor, so wird bei der Destillation auch nur wenig sekundäres tert-Butylamin der Formel I als Azeotrop mit Wasser ausgeschleust. Kleine Mengen an Azeotrop, beispielsweise solche, die weniger als ein Mol-% sekundäres tert.-Butylamin der Formel I, bezogen auf eingesetztes tert.-Butylamin, enthalten, können gegebenenfalls verworfen werden. Es ist aber auch möglich, das Azeotrop in die Natronlaugeextraktion zurückzuführen. Vorteilhaft ist, mit einer einstufigen Natronlaugebehandlung des Hydrieraustrags auszukommen. Außerdem muss keine Feinabstimmung der Natronlaugemenge erfolgen, um auch die letzten Reste an Wasser zu entfernen.

Der wasserfreie oder nur noch weniger als 5, bevorzugt weniger als 3, besonders bevorzugt weniger als 1 Gew.-% Wasser enthaltende Hydrieraustrag kann durch fraktionierende Destillation weiter aufgereinigt werden. Die Destillation kann kontinuierlich, bevorzugt diskontinuierlich durchgeführt werden. Dabei gehen in ersten Mischfraktionen, falls vorhanden, nicht umgesetztes tert.-Butylamin, nicht umgesetzter Aldehyd der Formel II und aus den Aldehyden entstandene Alkohole über Kopf ab. Dann folgt das erfindungsgemäße sekundäre tert.-Butylamin der Formel I, das ebenfalls über Kopf abdestilliert wird. Im Sumpf verbleiben, falls vorhanden, tertiäre Amine der Formel III und Hochsieder. Nicht ausreichend reine, Wertprodukte enthaltende Fraktionen, können in die Destillation zurückgeführt werden.

Für die fraktionierende Destillation kommen übliche Apparaturen in Betracht, wie sie beispielsweise in Kirk-Othmer, Encyclopedia of Chemical Technology, 3. Edition, Volume 7, John Wiley and Sons, New York,1979, Seiten 870 bis 881 beschrieben sind, wie Siebbodenkolonnen, Glockenbodenkolonnen, Packungskolonnen oder Füllkörperkolonnen.

Durch fraktionierende Destillation werden Reinheiten der sekundären tert.-Butylamine der Formel I von mehr als 98 FI.-%, insbesondere mehr als 99 FI.-%, besonders bevorzugt von mehr als 99,5 FI.-%, insbesondere mehr als 99,9 FI.-% erzielt (GC-Analyse)

Für die kontinuierliche Aufarbeitung nach EP-B 1.312.599 und EP-B 1.312.600 (Fig. 1 und 2) werden drei bis vier maßgeschneiderte Destillationskolonnen und eine Extraktionsvorrichtung benötigt.

Bei der bevorzugten erfindungsgemäßen diskontinuierlichen Aufarbeitung werden dagegen nur eine Destillationskolonne und eine Extraktionsvorrichtung eingesetzt.

### Beispiele

### Allgemeine Arbeitsvorschrift für die aminierende Hydrierung von Aldehyden mit tert.-Butylamin

Die Versuche wurden in einem 500-ml-Büchi-Laborautoklaven durchgeführt, der mit einem Scheiben-Rührer, Strömungsstörern und einer Zulaufpumpe ausgestattet war. In dem mit Stickstoff inertisierten Autoklaven wurden tert.-Butylamin und Hydrierkatalysator vorgelegt und unter Rühren auf die Reaktionstemperatur erwärmt. Der Reaktionsdruck wurde durch Aufpressen von Wasserstoff eingestellt. Innerhalb der jeweils angegebenen Zeit wurde der Aldehyd der Formel II zugepumpt. Im Falle von Acetaldehyd wurden das Vorratsgefäß und die Zulaufpumpe wegen des relativ hohen Acetaldehyd-Dampfdrucks gekühlt. Der Wasserstoffdruck wurde über ein RECO-Ventil konstant gehalten. Nachdem die Aldehyddosierung abgeschlossen war, wurde das Reaktionsgemisch unter den Reaktionsbedingungen 0,5 bis drei Stunden nachgerührt, dann abgekühlt und entspannt.

Der Hydrieraustrag wurde nach Abtrennung des Katalysators gaschromatographisch analysiert. Hierfür wurde eine GC-Säule RTX5-Amine verwendet (30 m, 0,32 mm, 1,5 µm, 60-5'-15-280/20, Helium). Die Analyseangaben stellen Flächenprozente dar.

### Vergleichsbeispiele 1 a bis 1 b und Beispiele 1 c bis 1 d

### Vergleich der Umsetzung von tert.-Butylamin, i-Propylamin und Ethylamin mit Acetaldehyd und Wasserstoff

Die Beispiele 1 a, 1 b und 1 c wurden mit jeweils 1,93 mol an Amin (Ethylamin, i-Propylamin bzw. tert.-Butylamin) bei 85 °C und 6 bar bzw. im Fall von Ethylamin bei 8 bar in Gegenwart eines Palladium-Katalysators (H0-50, 5 % Pd auf Kohle) durchgeführt. Das Molverhältnis von Aldehyd zu Amin betrug dabei 1 zu 1,05, die Menge an Hydrierkatalysator 2,0 Gew.-%, bezogen auf die Gesamtmenge an Amin. Der Acetaldehyd wurde dem Reaktor innerhalb von 6 Stunden zugeführt. Die Nachrührzeit betrug 1 Stunde bzw 2 Stunden (i-Propylamin).

In Beispiel 1 d wurde tert.-Butylamin mit Acetaldehyd in analoger Weise wie in Beispiel 1 c umgesetzt, jedoch bei einem Druck von 10 bar.

In Tabelle 2a sind die Umsetzungsbedingungen und Ausbeuten in Tabelle 2b die Ergebnisse der gaschromatographischen Analyse zusammengestellt.

Das erfindungsgemäße Beispiel 1 c zeigt, dass der Hydrieraustrag, wasserfrei gerechnet, zu > 94 FI.-% aus dem Zielprodukt Ethyl-tert.-butylamin besteht. Die Einsatzstoffe tert.-Butylamin und Acetaldehyd wurden bis auf 2,1 bzw. 0,2 FI.-% umgesetzt und die Menge an dem tertiären Diethyl-tert.-butylamin betrug nur 3 FI.-%.

Verwendet man dagegen gemäß den nicht erfindungsgemäßen Beispielen 1 b und 1 a anstelle von tert.-Butylamin iso-Propylamin bzw. Ethylamin, so sinkt die Menge an sekundärem iso-Propyl- bzw. Ethylamin überraschenderweise auf nur 70 FI.-% beziehungsweise nur 53 FI.-%. Parallel hierzu steigt die Menge an nicht umgesetztem Ausgangsamin deutlich und die Menge an unerwünschtem tertiärem Amin wesentlich an (Tabelle 2b).

Es ist überraschend, dass erfindungsgemäß gegenüber dem Stand der Technik ohne deutlichen Überschuss an Ausgangsamin (Tabelle 1), ohne Lösungsmittel und bei niedrigem Druck (Tabelle 1) mit 94 FI.-% höhere Ausbeuten erzielt werden. Weiterhin, dass bei niedrigem Druck ohne den Einsatz von wässriger Natronlauge (Tabelle 1) ähnlich hohe Ausbeuten möglich sind.

Das erfindungsgemäße Verfahren führt durch weitgehenden Umsatz der Ausgangskomponenten und geringe Bildung von tertiären Aminen der Formel III zu einer vereinfachten Aufarbeitung mit verringertem Energiebedarf bei hoher Reinheit des sekundären tert-Butylamins der Formel I.

Nicht vorherzusehen war auch, dass mit tert.-Butylamin erfindungsgemäß wesentlich höhere Ausbeuten an sekundären tert.-Butylaminen der Formel I als mit i-Propylamin und Ethylamin zu erzielen sind.

### Beispiele 2 a und 2 b

In analoger Weise wie in Beispiel 1 c wurden 1,5 mol tert.-Butylamin mit n-Propionaldehyd (Beispiel 2 a) bzw. n-Butyraldehyd (Beispiel 2 b) zu den entsprechenden sekundären Aminen (tert.-Butyl-Propyl-amin bzw. n-Butyl-tert.-Butyl-amin) umgesetzt und anschließend gaschromatographisch analysiert. Auch für diese Reaktionen konnten Ausbeuten von 93,6 FI.-% (Beispiel 2 a) bzw. 95.7 FI.-% (Beispiel 2 b) an den entsprechenden sekundären Aminen erzielt werden.

Für Beispiel 2 a wurden dabei gaschromatographisch die folgenden Nebenproduktanteile im Reaktionsgemisch festgestellt: Propionaldehyd (0,5 FI.-%), Propanol (0,6 FI.-%), tert.-Butylamin (0,7 FI.-%) und tertiäres Amin (1,3 FI.-%).

Und für Beispiel 2 b wurden dabei gaschromatographisch die folgenden Nebenproduktanteile im Reaktionsgemisch festgestellt: n-Butyraldehyd (0,4 FI.-%), n-Butanol (0,3 FI.-%), tert.-Butylamin (0,3 FI.-%) und tertiäres Amin (0,9 FI.-%).

### Beispiele 3 a und 3 b

In analoger Weise wie in Beispiel 1 c, jedoch unter der Verwendung von 4,0 Gew.-% Hydrierkatalysator, bezogen auf die Gesamtmenge an Amin, wurden 1 mol tert.-Butylamin mit Cyclohexanal (Beispiel 3 a) bzw. Benzaldehyd (Beispiel 3 b) zu den entsprechenden sekundären Aminen (tert-Butyl-cyclohexylmethyl-amin, bzw. Benzyl-tert-Butyl-amin) umgesetzt und anschließend gaschromatographisch analysiert. Für diese Reaktionen konnten Ausbeuten von 87,3 FI.-% (Beispiel 3 a) bzw. 14,2 FI.-% (Beispiel 3 b) an den entsprechenden sekundären Aminen erzielt werden.

In der gaschromatographische Analyse wurde für Beispiel 3 a das tert-Butyl-cyclohexylmethyl-imin (5,7 FI.-%) als hauptsächliches Nebenprodukt identifiziert. Es entstanden keine nachweisbaren Mengen des entsprechenden tertiären Amins. Die Menge des Zwischenprodukts tert-Butyl-cyclohexylmethyl-imin konnte durch die Verlängerung der Nachrührzeit um weitere 6 h auf einen Anteil von 2,3 FI.-% reduziert werden zugunsten der Ausbeute an tert-Butyl-cyclohexylmethyl-amin, die dabei auf 90,6 FI.-% anstieg.

Bei Beispiel 3b wurden in der gaschromatographischen Analyse als Nebenprodukte hauptsächlich unumgesetztes tert-Butylamin (26,1 FI.-%), Benzylalkohol (54 FI.-%) und Toluol (5,4 FI.-%) festgestellt. Es entstanden keine nachweisbaren Mengen des entsprechenden tertiären Amins. Durch Erhöhung der Reaktionstemperatur auf 100 °C konnte die Ausbeute an Benzyl-tert-Butyl-amin auf 19,6 FI.-% gesteigert werden bei gleichzeitiger Reduktion der Entstehung von Benzylalkohol. Durch weitere Erhöhung der Reaktionstemperatur auf 120 °C konnte die Ausbeute weiter auf 83,0 FI.-% gesteigert werden. Als Nebenprodukte wurden bei dieser Temperatur hauptsächlich unumgesetztes tert-Butylamin (3,4 FI.-%), Benzylalkohol (8,1 FI.-%) und Toluol (5,5 FI.-%) festgestellt.

**Tabelle 2a:**

| Beispiel Nr. | prim. Amin | Aldehyd | Molverhältnis Aldehyd / Amin | Katalysator | Zugabe | Zugabe-und Nachrührzeit [h] | Druck [bar] | LM bzw. Zusatz | Ausbeute |
|---|---|---|---|---|---|---|---|---|---|
| 1 a | Ethylamin | Acetaldehyd | 1 : 1.05 | H 0-50 (5 Gew.-%) | Amin vorgelegt | 7 (6+1) | 8 | - | 53 |
| 1 b | i-Propylamin | | | | | 8 (6+2) | 6 | - | 70 |
| 1 c | tert.-Butylamin | | | | | 7 (6+1) | 6 | - | 94 |
| 1 d | | | | | | 7 (6+1) | 10 | - | 94 |

**Tabelle 2b:**

| Beispiel Nr. | prim. Amin | Aldehyd | Hydrieraustrag (GC-Flächenprozent) | | | | | |
|---|---|---|---|---|---|---|---|---|
| | | | Acetaldehyd | Ethanol | Amin | sec. Amin I | tert. Amin III | Summe GC-FI.-% |
| 1 a | Ethylamin | Acetaldehyd | - | 0,01 | 7,9 | 52,9 | 28,3 | 89,1 |
| 1 b | iso-Propylamin | | 0,03 | - | 6,3 | 70,2 | 19,2 | 95,7 |
| 1 c | tert.-Butylamin | | 0,2 | 0,2 | 2,1 | 94,0 | 3,0 | 99,5 |
| 1 d | | | 0,4 | 0,6 | 0,4 | 94,3 | 3,4 | 99,1 |

## Patentansprüche

1. Verfahren zur Herstellung von unsymmetrischen sekundären tert-Butylaminen der Formel I durch reduktive Aminierung von Aldehyden der Formel II mit tert.-Butylamin und Wasserstoff in der Flüssigphase in Gegenwart von Hydrierkatalysatoren, umfassend folgende Schritte:
(i) Bereitstellung von tert-Butylamin und dem Hydrierkatalysator in einem Druckgefäß,
(ii) Zugabe von Wasserstoff und kontinuierliche Zugabe eines Aldehyds der Formel II
R-CHO II,
wobei der Aldehyd der Formel II ausgewählt ist aus der Gruppe von Acetaldehyd, Propionaldehyd, n-Butyraldehyd, i-Butyraldehyd, sekundärer Butyraldehyd, Pivalinaldehyd, n-Pentanat, n-htexanal, 2-Ethylhexanal, 2-Methylpentanal, 3-Methylpentanal, 4-Methylpentanal, n-Octanal, n-Decanal, n-Undecanal, n-Dodecanal, 11-Methyldodecanal, Cyclopentylaldehyd, Cyclohexylaldehyd, Cycloheptylaldehyd, Adamantylaldehyd, Phenylacetaldeyd und Benzaldehyd oder Gemische dieser Aldehyde und das Verhältnis zu Wasserstoff und Aldehyd der Formel II mindestens äquimolar ist,
(iii) Halten der Temperatur während der Zugabe des Aldehyds in Schritt (ii) im Bereich von 50 bis 150 °C und Halten des Gesamtdrucks während der Zugabe des Aldehyds in Schritt (ii) im Bereich von 2 bis 120 bar,
(iv) Entwässerung des Hydrieraustrag aus Schritt (iii), der das gebildete
sekundäre tert-Butylamin der Formel I 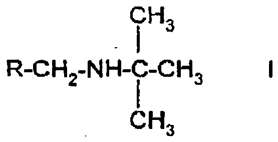 und das Reaktionswasser enthält und (v) anschließende fraktionierte Destillation des entwässerten Hydrieraustrages
aus Schritt (iv) und
wobei die Schritte (i) bis (iii) in Gegenwart eines unter den Reaktionsbedingungen inerten oder keines Lösungsmittels durchgeführt werden.

2. Verfahren nach Anspruch 1, wobei das Molverhältnis von in Schritt (i) eingesetztem tert.-Butylamin zu in Schritt (ii) zugeführtem Aldehyd der Formel II im Bereich von 1 zu 1 bis 1 zu 1,4 liegt.

3. Verfahren nach einem der Ansprüche 1 bis 2, wobei die Zuführung des Aldehyds der Formel II in Schritt (ii) innerhalb von 30 bis 600 Minuten erfolgt.

4. Verfahren nach einem der Ansprüche 1 bis 3, wobei die katalytisch aktive Masse des Hydrierkatalysators Metalle und/oder Metalloxide ausgewählt aus der Gruppe von Nickel, Kobalt, Ruthenium, Rhodium, Palladium, Platin, Kupfer und Gemischen dieser Metalle und/oder Metalloxide enthält.

5. Verfahren nach einem der Ansprüche 1 bis 4, wobei die Hydrierkatalysatoren im Fall einer Suspensionshydrierung aus dem Hydrieraustrag vor der Entwässerung in Schritt (iv) abgetrennt und in die Hydrierstufe in Schritt (i) zurückgeführt werden.

6. Verfahren nach einem der Ansprüche 1 bis 5, wobei die Entwässerung des katalysatorfreien Hydrieraustrags mit Hilfe von wässriger Natronlauge erfolgt.

7. Verfahren nach einem der Ansprüche 1 bis 5, wobei die Entwässerung des katalysatorfreien Hydrieraustrags durch Azeotropdestülation mit Kohlenwasserstoffen erfolgt.

8. Verfahren nach einem der Ansprüche 1 bis 7, wobei das Verfahren halbkontinuierlich betrieben wird.

## Claims

1. A process for preparing unsymmetric secondary tertbutylamines of the formula I by reductively aminating aldehydes of the formula II with tert-butylamine and hydrogen in the liquid phase in the presence of hydrogenation catalysts, comprising the following steps:
(i) providing tert-butylamine and the hydrogenation catalyst in a pressure vessel,
(ii) adding hydrogen and continuously adding an aldehyde of the formula II
R-CHO II
where the aldehyde of the formula II is selected from the group of acetaldehyde, propionaldehyde, n-butyraldehyde, i-butyraldehyde, sec- butyraldehyde, pivalaldehyde, n-pentanal, n-hexanal, 2-ethylhexanal, 2-methylpentanal, 3-methylpentanal, 4-methylpentanal, n-octanal, n-decanal, n-undecanal, n-dodecanal, 11-methyldodecanal, cyclopentylaldehyde, cyclohexylaldehyde, cycloheptylaldehyde, adamantylaldehyde, phenylacetaldehyde and benzaldehyde or mixtures of these aldehydes, and the ratio to hydrogen and aldehyde of the formula II is at least equimolar,
(iii) keeping the temperature during the addition of the aldehyde in step (ii) within the range from 50 to 150°C and keeping the total pressure during the addition of the aldehyde in step (ii) within the range from 2 to 120 bar,
(iv) dewatering the hydrogenation output from step (iii) which comprises the secondary tert-butylamine which is formed and is of the formula I 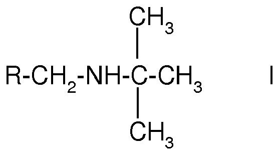 and the water of reaction, and
(v) subsequently fractionally distilling the dewatered hydrogenation output from step (iv) and
wherein steps (i) to (iii) are performed in the presence of a solvent which is inert under the reaction conditions or without solvent.

2. The process according to claim 1, wherein the molar ratio of tert-butylamine used in step (i) to aldehyde of the formula II supplied in step (ii) is in the range from 1:1 to 1:1.4.

3. The process according to either of claims 1 and 2, wherein the aldehyde of the formula (II) is supplied in step (ii) within 30 to 600 minutes.

4. The process according to any of claims 1 to 3, wherein the catalytically active material of the hydrogenation catalyst comprises metals and/or metal oxides selected from the group of nickel, cobalt, ruthenium, rhodium, palladium, platinum, copper and mixtures of these metals and/or metal oxides.

5. The process according to any of claims 1 to 4, wherein the hydrogenation catalysts, in the case of a suspension hydrogenation, are removed from the hydrogenation output before the dewatering in step (iv) and recycled into the hydrogenation stage in step (i).

6. The process according to any of claims 1 to 5, wherein the catalyst-free hydrogenation output is dewatered with the aid of aqueous sodium hydroxide solution.

7. The process according to any of claims 1 to 5, wherein the catalyst-free hydrogenation output is dewatered by azeotropic distillation with hydrocarbons.

8. The process according to any of claims 1 to 7, which is operated semicontinuously.

## Revendications

1. Procédé de fabrication de tert.-butylamines secondaires asymétriques de formule I par amination réductrice d'aldéhydes de formule II avec de la tert.-butylamine et de l'hydrogène en phase liquide en présence de catalyseurs d'hydrogénation, comprenant les étapes suivantes :
(i) la préparation de tert.-butylamine et du catalyseur d'hydrogénation dans un récipient sous pression,
(ii) l'ajout d'hydrogène et l'ajout continu d'un aldéhyde de formule II
R-CHO II
l'aldéhyde de formule II étant choisi dans le groupe constitué par l'acétaldéhyde, le propionaldéhyde, le n-butyraldéhyde, l'i-butyraldéhyde, le butyraldéhyde secondaire, le pivalinaldéhyde, le n-pentanal, le n-hexanal, le 2-éthylhexanal, le 2-méthylpentanal, le 3-méthylpentanal, le 4-méthylpentanal, le n-octanal, le n-décanal, le n-undécanal, le n-dodécanal, le 11-méthyldodécanal, le cyclopentylaldéhyde, le cyclohexylaldéhyde, le cycloheptylaldéhyde, l'adamantylaldéhyde, le phénylacétaldéhyde et le benzaldéhyde ou les mélanges de ces aldéhydes, et le rapport entre l'hydrogène et l'aldéhyde de formule II étant au moins équimolaire,
(iii) le maintien de la température pendant l'ajout de l'aldéhyde à l'étape (ii) dans la plage allant de 50 à 150 °C et le maintien de la pression totale pendant l'ajout de l'aldéhyde à l'étape (ii) dans la plage allant de 2 à 120 bar,
(iv) la déshydratation de la sortie d'hydrogénation de l'étape (iii), qui contient la tert.-butylamine secondaire de formule I formée 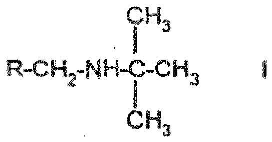 et l'eau de réaction, puis
(v) la distillation fractionnée de la sortie d'hydrogénation déshydratée de l'étape (iv),
les étapes (i) à (iii) étant réalisées en présence d'un solvant inerte dans les conditions de réaction ou sans solvant.

2. Procédé selon la revendication 1, dans lequel le rapport molaire entre la tert.-butylamine utilisée à l'étape (i) et l'aldéhyde de formule (II) introduit à l'étape (ii) se situe dans la plage allant de 1 sur 1 à 1 sur 1,4.

3. Procédé selon l'une quelconque des revendications 1 à 2, dans lequel l'introduction de l'aldéhyde de formule II à l'étape (ii) a lieu en 30 à 600 minutes.

4. Procédé selon l'une quelconque des revendications 1 à 3, dans lequel la masse catalytiquement active du catalyseur d'hydrogénation contient des métaux et/ou des oxydes de métaux choisis dans le groupe constitué par le nickel, le cobalt, le ruthénium, le rhodium, le palladium, le platine, le cuivre et les mélanges de ces métaux et/ou oxydes de métaux.

5. Procédé selon l'une quelconque des revendications 1 à 4, dans lequel, dans le cas d'une hydrogénation en suspension, les catalyseurs d'hydrogénation sont séparés de la sortie d'hydrogénation avant la déshydratation à l'étape (iv) et recyclés dans l'étape d'hydrogénation à l'étape (i).

6. Procédé selon l'une quelconque des revendications 1 à 5, dans lequel la déshydratation de la sortie d'hydrogénation sans catalyseur a lieu à l'aide de soude caustique aqueuse.

7. Procédé selon l'une quelconque des revendications 1 à 5, dans lequel la déshydratation de la sortie d'hydrogénation sans catalyseur a lieu par distillation azéotropique avec des hydrocarbures.

8. Procédé selon l'une quelconque des revendications 1 à 7, dans lequel le procédé est réalisé de manière semi-continue.
